# EUROPÄISCHE PATENTANMELDUNG

(11) **EP 0 605 894 A1**
(43) Veröffentlichungstag der Anmeldung: **13.07.1994**
(21) Anmeldenummer: 93121078.5
(22) Anmeldetag: 29.12.1993
(51) Int. Cl.: C12Q 1/00, C12Q 1/54, C12Q 1/26, C12Q 1/28, G01N 27/327

(54) **Analysenvorrichtung zur Bestimmung des Laktat- bzw. Glukosegehaltes**

(30) Priorität: 08.01.1993 DE 4300362
(71) Anmelder: Willms, Joachim, D-59519 Möhnesee (DE)
(72) Erfinder: Willms, Joachim, D-59519 Möhnesee (DE)
(74) Vertreter: Müller-Boré & Partner Patentanwälte

(57) **Zusammenfassung**

Gezeigt wird eine Analysenvorrichtung zur enzymatischen Bestimmung des Laktat- bzw. Glukosegehaltes einer Probe, mit einer Durchflußmeßzelle (4), die einen Eingangskanal (16) zur Einführung der zu analysierenden Probe und einen Ausgangskanal (14) zur Abführung der gemessenen Probe aufweist, wobei die Durchflußmeßzelle mindestens einen weiteren Anschlußkanal (18),(20) aufweist, der zur Einleitung einer bestimmten Flüssigkeit, insbesondere zur Verdünnung, Pufferung, Spülung oder Reaktionseinleitung dient.

## Beschreibung

Die vorliegende Erfindung betrifft eine Analysenvorrichtung zur enzymatischen Bestimmung des Laktat- bzw. Glukosegehaltes einer Probe, insbesondere einer Blutprobe gemäß dem Oberbegriff des Anspruchs 1.

Derartige Analysenvorrichtungen sind beispielsweise bekannt aus der DE 26 59 071 A1, der WO 89/01047 A1 sowie aus "Biosensors, A Practical Approach; Ed. A. E. G. Cass; Oxford University Press 1990, S. 10-11. Diese Druckschriften erlauben jedoch keine Probenverdünnung und bedürfen einer relativ hohen Enzymeinsatzmenge.

Derartige Analysenvorrichtungen dienen insbesondere zur automatisierten Bestimmung von Glukose bzw. Laktat im Vollblut, Serum oder Plasma. Durch den Einsatz von Enzymelektroden sind optimierte Durchflußzellen mit extrem kleiner Meßkammergeometrie geschaffen worden, die eine Bestimmung bei
hohen Probenraten und geringem Probenverbrauch ermöglichen.

Die Bestimmung der Glukose mit Hilfe einer Enzymelektrode erfolgt nach dem enzymatisch-amperometrischen Meßprinzip, bei dem die Probe durch den Eingangskanal in die Meßkammer gelangt und durch den Ausgangskanal aus der Meßkammer abgeführt wird. Die Meßkammer ist auf einer Seite durch die Enzymmembran begrenzt, in der das immobilisierte Enzym Glukoseoxydase (GOD) enthalten ist. Der Meßzyklus beginnt mit dem Eintauchen der Probenkanüle in eine Probe, wobei die Probenlösung durch den Eingangskanal in die Meßkammer geleitet wird. Die nach der Probennahme in die Meßkammer gelangte Glukose trifft nach Durchtritt durch die erste Membrangrenzschicht auf die immobilisierte Glukoseoxydase und wird mit Hilfe deren katalytischer Wirkung in Glukonsäure und Wasserstoffperoxid umgesetzt. Nach Diffusion des Wasserstoffperoxids durch die zweite Membrangrenzschicht wird das Wasserstoffperoxid an einer Elektrode, bevorzugt Platinelektrode bei einer bestimmten Spannung oxidiert, wobei Protonen, Sauerstoff und Elektronen erzeugt werden. Die erzeugten Elektronen führen zu einem Meßsignal an der Elektrode, das während der Probennahme fortlaufend nach der Zeit differenziert wird. Das Maximum der differenzierten Kurve kennzeichnet den maximalen Anstieg des Meßsignals (Wendepunkt). Das zu diesem Maximum gehörige Meßsignal wird in einen Spannungswert umgewandelt, der zu der Glukosekonzentration in der Probe proportional ist. Nach Erhalt des beschriebenen Meßsignals wird die Probennahme beendet. Nach Absinken des Meßsignals auf einen festgelegten Schwellwert in der Nähe einer Basislinie beginnt der nächste Meßzyklus.

Die Messung von Laktat geschieht mit derselben Durchflußmeßzelle, in deren Membran jedoch das immobilisierte, aktive Enzym Laktatooxydase (LOD) enthalten ist. Durch Einwirkung dieses Enzyms wird Laktat katalytisch zu Pyruvat und Wasserstoffperoxid oxidiert. Das Wasserstoffperoxid wird in gleicher Weise, wie oben beschrieben, nachgewiesen.

Auf der Basis dieses Meßprinzips sind bereits Analysenvorrichtungen bekannt, die verschiedene Proben durch die genannten enzymatischen Reaktionen bestimmen, in deren Folge an einer Indikatorelektrode eine Potentialänderung bzw. Stromänderung entsteht.

Es sind Geräte bekannt, die eine relativ große Meßkammer (z. B. Volumina von 1 ml) mit einer Öffnung zum Einpipettieren der Proben und einer Mischeinrichtung, z. B. einem Rührer aufweisen, der zur intensiven Durchmischung der Probenflüssigkeit mit der in der Meßkammer befindlichen Verdünnungsflüssigkeit oder Enzymlösung dient.

Unter diesen Geräten gibt es Ausführungen, bei denen sich das zur Reaktion benötigte Enzym im gelöster Form in der Meßkammer befindet (beispielsweise der Glukose-Analysator der Firma Beckmann, USA), und es gibt Ausführungen, bei denen das zur Reaktion benötigte Enzym in immobilisierter Form in einem Reaktor (Glukose-Analysator der Firma APEC, USA) oder in einer doppelwandigen Enzymmembran befindet, die die Elektrode überspannt (z. B. YSI 23 der Firma Yellow Springs Instruments, USA).

Ferner sind Meßzellen bekannt, die als Durchflußmeßzellen mit einem Eingang zur Zuführung der Probe und Spüllösung und einem Ausgang zur Abfuhr der gemessenen Probe bzw. Abfallflüssigkeit versehen sind. Derartige Meßzellen weisen ebenfalls eine in die Meßkammer ragende membranüberspannte Meßelektrodenanordnung auf, z. B. ESAT 6660 der Firma Prüfgeräte-Werk Medingen GmbH, Deutschland. Diese Meßzellen zeichnen sich gegenüber den gerührten Meßzellen durch ein extrem kleines Meßkammervolumen von wenigen µl aus. Bei diesen Zellen erfolgt die Zufuhr sowohl der Probe als auch der Spülflüssigkeit zeitlich nacheinander durch einen Eingangskanal, wobei zumeist an den Ausgangskanal eine Saugpumpe angeschlossen ist.

Die beschriebenen Anordnungen haben verschiedene Nachteile. So benötigen die oben beschriebenen gerührten Meßzellen im allgemeinen einen zeitaufwendigen und flüssigkeitsaufwendigen Spülprozess zwischen der Messung zweier Proben, der zu verhältnismäßig geringen Probenraten führt.

Die beschriebenen Durchflußmeßzellen können demgegenüber keinen Verdünnungsschritt in der Meßkammer realisieren und benötigen beim Einsatz von gelösten Enzymen eine größere Enzymmenge, da auch während der Spülzeit Enzyme gefördert werden müssen. Schließlich werden die mit Enzymmembranen arbeitenden Meßzellen in ihrer Meßgeschwindigkeit und somit in ihrer Probenrate durch die Diffusionsgeschwindigkeit der Glukosemoleküle durch die Membranen begrenzt.

Aufgabe der vorliegenen Erfindung ist es daher, ein Analysengerät gemäß Oberbegriff des Anspruchs 1 derart weiterzubilden, daß eine Analysenvorrichtung geschaffen wird, die eine Probenverdünnung, ein Arbeiten mit gelöstem Enzym bei minimalem Enzymeinsatz und gleichzeitig eine hohe Probenrate aufgrund eines optimierten Spülsystems realisiert.

Diese Aufgabe wird erfindungsgemäß dadurch gelöst, daß die Durchflußmeßzelle mindestens einen weiteren Anschlußkanal aufweist, der zur Einleitung einer bestimmten Flüssigkeit, insbesondere zur Verdünnung, Pufferung, Spülung oder Reaktionseinleitung dient.

Der Erfindung liegt die Idee zugrunde, mehrere Flüssigkeitsströme, die zur Verdünnung, zur Ingangsetzung einer chemischen Reaktion oder eines Spülvorgangs in der Meßkammer einer Durchflußmeßzelle dienen, direkt in der Meßkammer gleichzeitig oder zeitlich aufeinanderfolgend zusammenzuführen. Dadurch kann eine solche Anordnung unter sparsamer Verwendung gelöster Enzyme betrieben werden und kann ebenfalls die Nachteile vermeiden, die sich durch das Hindurchleiten des Probenvolumens, das sich zwangsläufig nach Erreichen des Meßwertes noch im Probenzuführungskanal befindet, ergeben. Somit werden die geschwindigkeitsbegrenzenden Ursachen Diffusion der Glukose durch die erste Membranschicht und Einleiten von Probe nach Erreichen des Meßwertes, die im Stand der Technik zu geringen Probenraten führen, vermieden.

Es ist bevorzugt, daß die Durchflußmeßzelle eine Meßkammer aufweist, an deren Wandung die Anschlüsse des Eingangskanals, des Ausgangskanals und des weiteren Anschlußkanals gebildet sind. Die Anschlüsse befinden sich bevorzugt in einer Wandung der Meßkammer, sodaß eine geeignete Vermischung bzw. Verwirbelung der zugeführten Flüssigkeiten gewährleistet ist.

Bevorzugt definieren die Anschlüsse einen in einer Ebene liegenden Anschlußbereich in der Meßkammer. Auch diese Ausgestaltung gewährleistet eine besonders wirksame Vermischung der eingeführten Flüssigkeiten.

Die dem Anschlußbereich gegenüberliegende Wand der Meßzelle wird bevorzugt durch eine Membran gebildet. Hierbei kann es sich beispielsweise um eine oben genannte Enzymmembran handeln, die ein immobilisiertes Enzym enthält oder um eine Membran, die kein Enzym enthält. Da die Membran zum Durchtritt von Glukose bzw. Laktat dient, ist eine Führung der durch die Kanäle geführten Flüssigkeit in Richtung auf die Membran vorteilhaft.

In einer ersten bevorzugten Ausführungsform sind zwei Anschlußkanäle gebildet, die in der Meßkammer münden. Die beiden Anschlußkanäle dienen besonders bevorzugt zur Zuführung einer Verdünnungslösung und einer Enzymlösung. Auf diese Weise kann ein immoblisiertes Enzym in der Membran entfallen und die Verdünnungsflüssigkeit bzw. Enzymlösung kann zeitlich gleichzeitig mit der Probenlösung zugeführt werden.

Es ist bevorzugt, daß die Mittelpunkte der Kanäle im wesentlichen die Eckpunkte eines Quadrates bilden. Auf diese Weise haben jeweils zwei benachbarte Kanäle denselben Abstand und eine gleichmäßige Vermischung ist garaniert. Der Abstand der Mittelpunkte der Kanäle im Anschlußbereich voneinander liegt bevorzugt im Bereich des 1,2- bis 2,5fachen des Durchmessers der Kanäle. Bei dieser Dimensionierung wird eine besonders vorteilhafte Verwirbelung beobachtet. Der Abstand zwischen dem Anschlußbereich und der Membran ist bevorzugt gleich oder kleiner als der Durchmesser der Kanäle. Auf diese Weise kann auch bei geringen Fördergeschwindigkeiten bzw. Fördervolumina durch die Kanäle eine sofortige gleichzeitige Benetzung der Membran durch die in den Kanälen geführten Flüssigkeiten erreicht werden.

Die Kanäle sind bevorzugt zumindest abschnittsweise gradlinig zum Anschlußbereich in der Meßkammer geführt, wobei die Mittelachsen der Kanäle in den gradlinigen Abschnitten sowohl gegenüber einer horizontalen als auch gegenüber einer vertikalen Ebene geneigt sind. Dabei sind gegenüberliegende Kanäle bevorzugt derart gegensinnig geneigt, daß gedachte Verlängerungen der Kanäle sich im Bereich der Membran oder dahinter schneiden. Auch diese Maßnahme gewährleistet, daß ein unmittelbares Zusammentreffen der Flüssigkeiten auf der Membranoberfläche gewährleistet ist, um den Meßvorgang abzukürzen. Der Eingangskanal ist bevorzugt mit einer Mischeinrichtung verbunden, die mit einer Dosiereinrichtung in Verbindung steht. Die Mischeinrichtung dient zur geeigneten Vermischung des entnommenen Serums mit anderen Flüssigkeiten. Die Dosiereinrichtung dient zur zeitlich gesteuerten Zuführung der zu analysierenden Probe.

In einer zweiten Ausführungsform ist ein Anschlußkanal gebildet, der in der Meßkammer mündet. Dieser Anschlußkanal dient bevorzugt zur Einleitung einer Verdünnungslösung in die Meßkammer, wobei ebenfalls bevorzugt ein immobilisiertes Enzym in der Membran vorhanden ist.

In dieser Ausführungsform verbindet der Anschlußkanal die Meßkammer mit einem Behälter für Pufferlösung, so daß die Verdünnungslösung schnell gefördert werden kann. Zwischen der Meßkammer und dem Behälter ist bevorzugt ein Thermostat angeordnet, um die Temperatur geeignet einzustellen. Ferner ist zwischen Meßkammer und Behälter bevorzugt ein Ventil angeordnet, das zur Steuerung der Pufferlösungsentnahme dient.

Der Eingangskanal ist bevorzugt mit einer Mischeinrichtung verbunden, die mit einer Dosiereinrichtung in Verbindung steht. Die Mischeinrichtung dient zur geeigneten Vermischung des entnommenen Serums mit anderen Flüssigkeiten. Die Dosiereinrichtung dient zur zeitlich gesteuerten Entnahme, Aufbereitung und Abgabe der entnommenen Probe. Der Eingangskanal ist in gleicher Weise gestaltet wie bereits oben beschrieben.

Im Eingangskanal ist bevorzugt zwischen Meßkammer und Mischeinrichtung ein Sperrventil angeordnet, das zur zeitlich gesteuerten Zuführung der zu analysierenden Probe dient.

Von dem Eingangskanal zweigt bevorzugt eine Leitung ab, die mit einer Pumpe verbunden ist, um nicht mehr gebrauchte Probenlösung abzupumpen. In der Leitung ist bevorzugt ein Ventil angeordnet, um die Absaugung zeitlich zu steuern.

Es ist bevorzugt, daß in dieser zweiten Ausführungsform die Membran ein immobilisiertes Enzym enthält.

Weitere Vorteile, Merkmale und Anwendungsmöglichkeiten der vorliegendenden Erfindung ergeben sich aus der nachfolgenden Beschreibung bevorzugter Ausführungsbeispiele in Verbindung mit der Zeichnung.

Figur 1 zeigt eine Seitenansicht der ersten Ausführungsform der vorliegenden Erfindung mit zwei zusätzlichen Anschlußkanälen.

Figur 2 zeigt eine Rückansicht der Ausführungsform von Figur 1.

Figur 3 zeigt eine Aufsicht auf die Ausführungsform von Figur 1.

Figur 4 zeigt eine prinzipielle Flüssigkeitsschaltung für die Analysenvorrichtung gemäß Figur 1.

Figur 5 zeigt eine prinzipielle Flüssigkeitsschaltung für eine Analysenvorrichtung gemäß der zweiten Ausführungsform mit einem zusätzlichen Anschlußkanal.

Figur 6 zeigt eine Flüssigkeitsschaltung der zweiten Ausführungsform gemäß Figur 5.

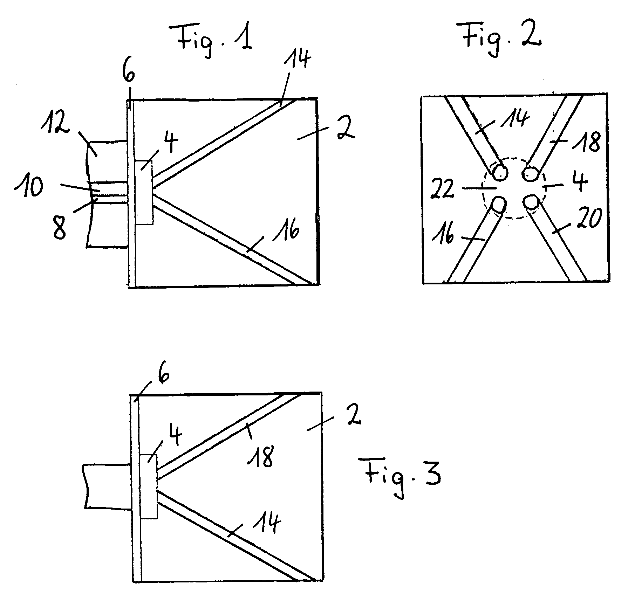

Die in den Figuren 1, 2 und 3 beschriebene erste Ausführungsform der erfindungsgemäßen Analysenvorrichtung weist eine Durchflußmeßzelle 2 auf, die beispielsweise aus einem Kunststoff-Spritzformteil besteht. Die Durchflußmeßzelle 2 weist einen Eingangskanal 16, einen Ausgangskanal 14 sowie einen zur Zuführung von Verdünnungslösung bestimmten Anschlußkanal 20 und einen zur Zuführung von Enzymlösung bestimmten Anschlußkanal 18 auf. Die Kanäle 14, 16, 18 und 20 sind zu einer kreiszylindrisch ausgebildeten Meßkammer 4 geführt, wobei die Anschlüsse der Kanäle 14, 16, 18, 20 einen in einer Wandungsebene gelegenen Anschlußbereich 22 bilden. Die dem Anschlußbereich 22 gegenüberliegende Kammerwandung wird durch eine Membran 6 gebildet, hinter welcher eine Indikatorelektrode 8 angeordnet ist, die zum Nachweis des durch die Wandung tretenden Wasserstoffperoxids dient. Die Indikatorelektrode ist benachbart der hinteren Membranwand der Membran 6 angeordnet, so daß eine unmittelbare Oxidation des Wasserstoffperoxids gewährleistet ist. Benachbart der Indikatorelektrode 8 ist ein Isolationsmaterial 10, das die Indikatorelektrode 8 von einer Referenzelektrode 12 trennt, die als Bezugselektrode dient.

Die vier Kanäle 14, 16, 18, 20 sind, wie in Figur 2 gezeigt, gegenüber einer horizontalen Ebene und einer vertikalen Ebene derart geneigt, daß sie strahlenförmig divergieren. Dabei ist der Abstand jeweils der Mittelpunkte benachbarter Kanäle 14, 18 bzw. 18, 20 bzw. 20, 16 bzw. 16, 14 derart ausgestaltet, daß dieser Abstand das 1,2- bis 2,5fache des Durchmessers eines Kanals im Anschlußbereich beträgt. Ferner ist der axiale Durchmesser der Meßkammer 4, d. h. der Abstand zwischen dem Anschlußbereich 22 der einen Wandung und der Membran 6 so ausgebildet, daß er kleiner oder gleich ist dem Durchmesser der Kanäle, wobei die Kanäle im wesentlichen gleichen Durchmesser haben. Die Meßkammer 4 hat somit eine geringe axiale Breite. Die einzelnen Kanäle sind derart geneigt, daß sie in gedachter Verlängerung konvergieren, und zwar im Bereich der Membran 6, so daß die durch die Kanäle geförderten Flüssigkeiten sich im Bereich der Membran 6 treffen und vermischen.

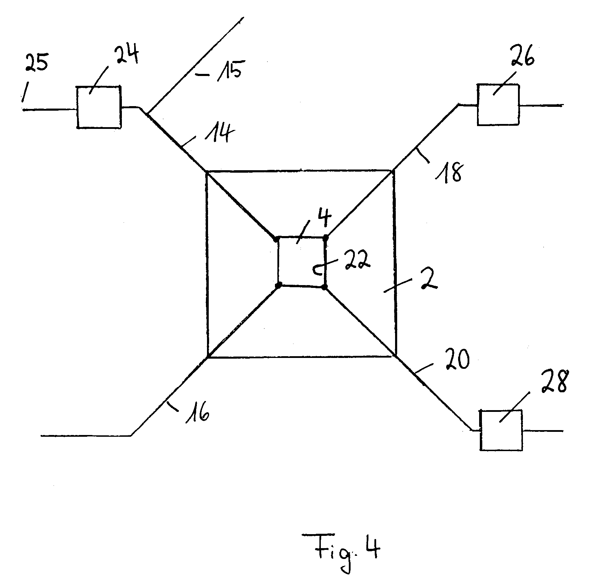

In Figur 4 ist eine Flüssigkeitsschaltung zum Betrieb der in den Figuren 1 bis 3 erläuterten Durchflußmeßzelle gezeigt. Durch die Eingangsleitung 16 wird eine Probenflüssigkeit bzw. Spülflüssigkeit vom Probennehmer in die Meßkammer 4 geleitet, die speziell in diesem Ausführungsbeispiel quadratischen Querschnitt hat. Die verbrauchte Probe bzw. Spülflüssigkeit wird durch den Ausgangskanal 14 abgesaugt, wobei ein Ventil 24 in dem Ausgangskanal 14 vorgesehen ist. Ferner zweigt von dem Ausgangskanal 14 eine Leitung 15 ab, die somit direkt aus der Meßkammer 4 ohne Ventil abziehen kann. Ferner ist ein Anschlußkanal 18 vorgesehen, der zur Zuführung von Enzymlösung dient und der ein Ventil 26 aufweist. Schließlich ist ein Anschlußkanal 20 zur Zuführung von Verdünnungslösung vorgesehen, der ein Ventil 28 aufweist.

Die Flüssigkeitsschaltung nach Figur 4 wird folgendermaßen betrieben. Zunächst wird ein Meßzyklus ohne Probenverdünnung beschrieben, bei dem das Ventil 24 und die daran anschließende Leitung 25 nicht vorhanden sind. Mit der Leitung 15 ist eine Pumpe verbunden, die durch die Meßkammer 4 ansaugt. Die Pumpe saugt durch die Eingangsleitung 16 und/oder durch die Anschlußleitung 20 an. Die Fördermengen sind dabei nahezu gleichgroß, wobei durch die Stellung des Ventils 28 die Zuführung von Verdünnungslösung gesteuert werden kann. Bei dem nun folgenden Spülvorgang wird zunächst Spülflüssigkeit von der an Leitung 15 angeschlossenen Pumpe durch den Eingangskanal 16 gefördert. Ferner wird zusätzlich die Verdünnungslösung über einen separaten Pumpkanal, der ein kleineres Fördervolumen realisiert als der absaugende Kanal in die Meßkammer 4 gedrückt, da das Ventil 28 geöffnet ist. Das Ventil 26 ist geschlossen, so daß keine Enzymlösung gefördert wird. Bei dem nun folgenden Meßvorgang wird durch den Eingangskanal 16 Probenlösung gefördert, wobei das Ventil 26 geöffnet ist, so daß ebenfalls Enzymlösung in die Meßkammer gedrückt wird. Das Ventil 28 ist geschlossen, so daß keine Verdünnungslösung gefördert wird. Die Fördermenge der Enzymlösung entspricht dabei etwa der der Verdünnungsflüssigkeit.

Ein Betriebszyklus ohne Probenverdünnung, bei dem die Leitung 15 nicht vorhanden ist, ist so ausgestaltet, daß eine Pumpe an Leitung 25 hinter dem Ventil 24 angeschlossen ist und saugt. Zum Messen, d. h. wenn Probe angesaugt wird, ist das Ventil 26 geöffnet und es wird über einen weiteren Pumpkanal Enzymlösung in die Meßkammer gedrückt.

Das Ventil 24 ist geöffnet und da das Fördervolumen des an 25 angeschlossenen Pumpkanals größer ist als das Volumen der zugeführten Enzymlösung, wird diese und gleichzeitig die Probe, die über Kanal 16 in die Meßkammer gelangt, angesaugt. Nach Erreichen des Meßwertes werden die Ventile 24 und 26 geschlossen und das Ventil 28 wird geöffnet. Über Leitung 20 wird nun Spüllösung in die Meßkammer gedrückt, die über Leitung 16 abgeführt wird und damit sowohl aus der Meßkammer als auch aus der Leitung 16 die Probe hinausspült. Dabei muß sichergestellt sein, daß das Ende der Leitung 16 zu diesem Zeitpunkt in ein Abfallgefäß mündet.

Ein alternativer Betriebszyklus mit Probenverdünnung basiert auf einer Flüssigkeitsschaltung, bei der das Ventil 24 die Leitung 25 und die Leitung 15 vorhanden sind, jedoch das Ventil 28 in Kanal 20 entfällt.

Zur Messung wird über Kanal 16 an die Probelösung angeschlossen. Gleichzeitig werden die Ventile 26 und 24 geöffnet. Dadurch wird über Leitung 18 über einen weiteren Pumpkanal Enzymlösung in die Meßkammer gedrückt. Das gleiche Volumen, das über Leitung 18 zusätzlich zugeführt wird, wird über einen weiteren Pumpkanal, der an Leitung 25 angeschlossen ist, aus der Meßkammer abgesaugt. Zur Spülung saugt die Pumpe an Leitung 15 ein bestimmtes Flüssigkeitsvolumen an.

Gleichzeitig wird über Kanal 20 über einen weiteren Pumpkanal ein kleineres Volumen Verdünnungsflüssigkeit in die Meßkammer gedrückt. Die entstehende Volumendifferenz wird über Kanal 16, der zu dieser Zeit ebenfalls an die Spülflüssigkeit angeschlossen ist, angesaugt.

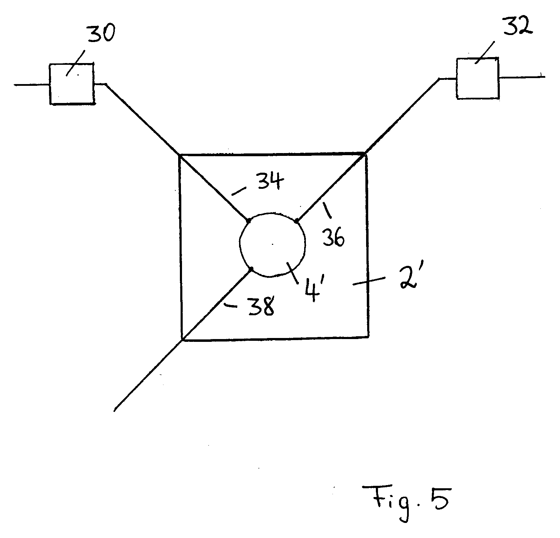

Die in Figur 5 gezeigte Flüssigkeitsschaltung beruht auf einer Ausführungsform mit nur einem Anschlußkanal 36, der zusammen mit einem Eingangskanal 38 und einem Ausgangskanal 34 in einer Meßkammer 4' einer Durchflußmeßzelle 2' mündet. In dieser Ausführungsform ist bereits ein immobilisiertes Enzym in einer die Meßkammer 4' abschließenden Membran enthalten, die ähnlich der Membran 6 von Figur 1 ausgestaltet ist.

Zunächst wird ein Meßzyklus ohne Probenverdünnung beschrieben, bei dem eine Pumpe an Kanal 34 angeschlossen ist und durch Kanal 38 Spülflüssigkeit ansaugt. Je nach Stellung des Ventils 32 wird durch Kanal 36 Verdünnungslösung in die Meßkammer 4' gefördert.

Beim Messen ist Ventil 32 geschlossen und Ventil 30 geöffnet. Die Pumpe saugt über Kanal 38 Probeflüssigkeit an. Nach Erreichen des Meßwertes wird die Spülung der Meßkammer wie folgt durchgeführt: Beim Spülen ist Ventil 30 geschlossen und Ventil 32 geöffnet. Über Kanal 36 wird Spülflüssigkeit in die Meßkammer 4 gedrückt, die diese über Kanal 38 wieder verläßt. Dabei wird die Meßkammer und der Kanal 38 gespült. Dieser muß zu dieser Zeit in ein Abfallgefäß münden.

Nachstehend wird ein Bertriebszyklus mit Probenverdünnung beschrieben, bei dem eine Pumpe an Kanal 34 angeschlossen ist und das Ventil 32 entfällt.

Beim Messen ist Ventil 30 geöffnet. Die Saugpumpe saugt über Kanal 34 sowohl die über einen weiteren Pumpkanal mit geringerem Fördervolumen über Kanal 36 in die Meßkammer gedrückte Verdünnungslösung als auch ein sich aus der Fördervolumendifferenz ergebendes Probenvolumen über Kanal 38 an. Nach Erreichen des Meßwertes beginnt die Spülung. Beim Spülen ist Ventil 30 geschlossen. Die über Kanal 36 in die Meßkammer gedrückte Verdünnungslösung verläßt diese über Kanal 38 und spült somit die Probe aus der Meßkammer und aus dem Kanal 38 heraus.

In allen Fällen übernimmt ein Steuerrechner die zeitliche Steuerung für den Wechsel von Spülvorgang und Meßvorgang, wobei der Ablauf entweder mit Festzeiten oder durch Schwellwerte gesteuert wird. Da beide Verfahren prinzipiell bekannt sind, wird auf eine nähere Erläuterung verzichtet. In einer weiteren Ausgestaltung wird die Probe in bekannter Weise durch einen Diluter in ein Gefäß verdünnt und dieses Gefäß in geeigneter Weise an den Probeneingang der Meßkammer angeschlossen. Die nachfolgend beschriebene Flüssigkeitsschaltung ist insbesondere für Meßkammern geeignet, in die keine Flüssigkeiten hineingedrückt werden können. Demzufolge darf nur eine Saugpumpe an die Meßkammer angeschlossen werden.

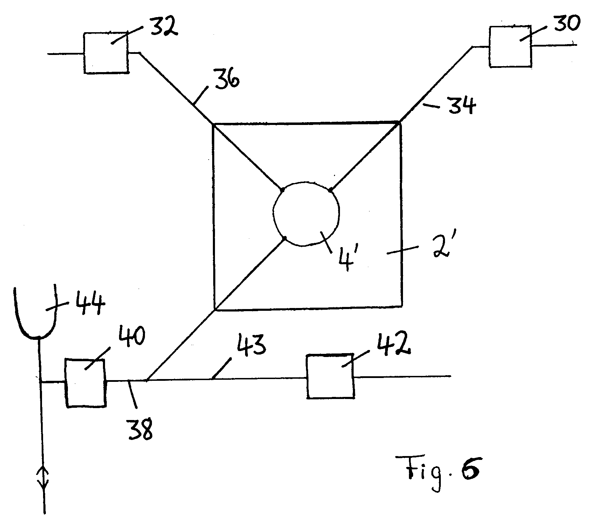

In Figur 6 ist eine erweiterte Flüssigkeitsschaltung der zweiten Ausführungsform nach Figur 5 gezeigt, wobei von dem Eingangskanal 38 eine Leitung 43 abzweigt, die über ein Ventil 42 mit einer Saugpumpe verbunden ist. Ferner ist die Eingangsleitung 38 über ein Ventil 40 mit einer Mischeinrichtung 44 verbunden, die mit einem Dosierer in Verbindung steht. Hinter der Mischeinrichtung 44 ist der Eingangskanal 38 mit einer zuschaltbaren Pumpe verbunden. Der Dosierer kann die Probe wahlweise auch mit Enzymlösung verdünnen. In diesem Fall kann ein Enzym in der Meßkammer entfallen.
1. Zusätzlich bzw. alternativ zu dem bereits vorstehend zu Figur 5 beschriebenen Betriebszyklus ist bei der Ausführungsform nach Figur 6 bevorzugt, daß zunächst die Ventile 40 und 30 geschlossen sind, während Ventile 32 und 42 geöffnet sind. Die Meßkammer wird somit mit Pufferlösung durch Kanal 36 gespült.
2. Nachfolgend wird Ventil 32 geschlossen, während Ventil 30 geschlossen bleibt und Ventil 40 wird geöffnet, während Ventil 42 geöffnet bleibt. Der Eingangskanal 38 wird bis zur Verzweigungsstelle der Leitung 43 mit Luft gefüllt, während die in der Leitung noch vorhandene alte Probenflüssigkeit durch die Leitung 43 abgesaugt wird.
3. Jetzt werden, wie zu 1. beschrieben, die Ventile 40 und 30 geschlossen, während die Ventile 32 und 42 geöffnet sind. Durch den Dosierer wird eine Probenflüssigkeit in die Mischeinrichtung 44 gegeben, wahlweise unverdünnt oder verdünnt.
4. Nun werden, wie zu 2. beschrieben, die Ventile 32 und 30 geschlossen, während die Ventile 40 und 42 geöffnet sind. Der Kanal 38 bis zur Verzweigungsstelle 43 wird mit Probenlösung gefüllt, wobei ein geringer Abfall (nicht zu vermeiden) durch Leitung 43 in den Abfall gesaugt wird, um eine Verschleppung zu vermeiden.
5. Nachfolgend werden die Ventile 40 und 30 geöffnet, während die Ventile 32 und 42 geschlossen sind. Die Probe wird gemessen.
6. Nun wird bei Erreichen des Maximums das Ventil 32 und das Ventil 42 geöffnet, die Ventile 40 und 30 geschlossen. Über die zuschaltbare Pumpe wird die Mischeinrichtung 44 entleert. Gleichzeitig erfolgt die Spülung der Meßzelle und des Abschnittes des Kanals 38 zwischen Verbindungsleitung 43 und Meßkammer 4'.
7. Es folgt der Vorgang nach 2.
8. Es folgt der Vorgang nach 3.
9. Es folgt der Vorgang nach 4.
10. Es wird abgewartet, bis die Kammerspülung abgeschlossen ist; die Meßkammer 2' nach Figur 6 enthält eine Enzymmembran wie oben beschrieben, dann wird weiter mit Schritt 5. verfahren.

In allen Fällen übernimmt ein Steuerrechner die zeitliche Steuerung für den Wechsel von Spülvorgang und Meßvorgang wobei der Ablauf entweder mit Festzeiten oder durch Schwellwerte gesteuert wird. Da beide Verfahren prinzipiell bekannt sind, wird auf eine nähere Erläuterung verzichtet. In einer weiteren Ausgestaltung wird die Probe in bekannter Weise durch einen Diluter in ein Gefäß verdünnt und dieses Gefäß in geeigneter Weise an den Probeneingang der Meßkammer angeschlossen.

Die Meßkammer 4' nach Figur 6 enthält eine Enzymmembran, die geeignet ist zur Bestimmung der Stoffwechselprodukte Glukose, Laktat, Harnsäure, Harnstoff und Cholesterin.

## Patentansprüche

1. Analysenvorrichtung zur enzymatischen Bestimmung des Laktat- bzw. Glukosegehaltes einer Probe, mit einer Durchflußmeßzelle (2, 2'; 4, 4'), die einen Eingangskanal (16; 38) zur Einführung der zu analysierenden Probe und einen Ausgangskanal (14; 34) zur Abführung der gemessenen Probe aufweist,
dadurch gekennzeichnet, daß die Durchflußmeßzelle (2, 2'; 4, 4') mindestens einen weiteren Anschlußkanal (18, 20; 36) aufweist, der zur Einleitung einer bestimmten Flüssigkeit, insbesondere zur Verdünnung, Pufferung, Spülung oder Reaktionseinleitung dient.

2. Analysenvorrichtung nach Anspruch 1, dadurch gekennzeichnet, daß die Durchflußmeßzelle (2, 2'; 4, 4') eine Meßkammer (4, 4') aufweist, an deren Wandung die Anschlüsse des Eingangskanals (16; 38), des Ausgangskanals (14; 34) und des weiteren Anschlußkanals (18, 20; 36) gebildet sind.

3. Analysenvorrichtung nach Anspruch 2, dadurch gekennzeichnet, daß die Anschlüsse (14, 16, 18, 20; 34, 36, 38) einen in einer Ebene liegenden Anschlußbereich (22) in der Meßkammer (4, 4') definieren.

4. Analysenvorrichtung nach Anspruch 3, dadurch gekennzeichnet, daß die dem Anschlußbereich (22) gegenüberliegende Wandung der Meßkammer (4, 4') durch eine Membran (6) gebildet ist.

5. Analysenvorrichtung nach Anspruch 1, dadurch gekennzeichnet, daß zwei Anschlußkanäle (18, 20) gebildet sind, die in der Meßkammer (4) münden.

6. Analysenvorrichtung nach Anspruch 5, dadurch gekennzeichnet, daß die beiden Anschlußkanäle (18, 20) zur Zuführung einer Verdünnungslösung (20) und einer Enzymlösung (18) dienen.

7. Analysenvorrichtung nach Anspruch 5 oder 6, dadurch gekennzeichnet, daß die Mittelpunkte der Kanäle (14, 16, 18, 20) im wesentlichen die Eckpunkte eines Quadrats bilden.

8. Analysenvorrichtung nach einem der vorhergehenden Ansprüche, dadurch gekennzeichnet, daß der Abstand der Mittelpunkte der Kanäle im Anschlußbereich (22) voneinander im Bereich des 1,2- bis 2,5fachen Durchmessers der Kanäle liegt.

9. Analysenvorrichtung nach Anspruch 4, dadurch gekennzeichnet, daß der Abstand zwischen dem Anschlußbereich (22) und der Membran (6) gleich oder kleiner ist als der Durchmesser der Kanäle.

10. Analysenvorrichtung nach einem der vorhergehende Ansprüche, dadurch gekennzeichnet, daß die Kanäle (14, 16, 18, 20; 34, 36, 38) zumindest abschnittsweise gradlinig zum Anschlußbereich (22) in der Meßkammer (4) geführt sind, wobei die Mittelachsen der Kanäle in den gradlinigen Abschnitten sowohl gegenüber einer horizontalen als auch gegenüber einer vertikalen Ebene geneigt sind.

11. Analysenvorrichtung nach Anspruch 10, dadurch gekennzeichnet, daß gegenüberliegende Kanäle derart gegensinnig geneigt sind, daß gedachte Verlängerungen der Kanäle sich im Bereich der Membran (6) oder dahinter schneiden.

12. Analysenvorrichtung nach Anspruch 1, dadurch gekennzeichnet, daß ein weiterer Anschlußkanal (36) gebildet ist, der in der Meßkammer (4') mündet.

13. Analysenvorrichtung nach Anspruch 12, dadurch gekennzeichnet, daß der Anschlußkanal (36) zur Einleitung einer Verdünnungslösung in die Meßkammer (4') dient.

14. Analysenvorrichtung nach Anspruch 12, dadurch gekennzeichnet, daß der Anschlußkanal (36) die Meßkammer (4') mit einem Behälter für Spüllösung verbindet.

15. Analysenvorrichtung nach einem der vorhergehenden Ansprüche, dadurch gekennzeichnet, daß alle Anschlußkanäle und der Eingangskanal jeweils einen Thermostaten aufweisen.

16. Analysenvorrichtung nach Anspruch 14, dadurch gekennzeichnet, daß zwischen Meßkammer (4') und Behälter ein Ventil (32) angeordnet ist.

17. Analysenvorrichtung nach Anspruch 12, dadurch gekennzeichnet, daß der Eingangskanal (38) mit einer Mischeinrichtung (44) verbunden ist, die mit einer Dosiereinrichtung in Verbindung steht.

18. Analysenvorrichtung nach Anspruch 17, dadurch gekennzeichnet, daß im Eingangskanal (38) zwischen Meßkammer (4') und Mischeinrichtung (44) ein Sperrventil (40) angeordnet ist.

19. Analysenvorrichtung nach Anspruch 12, dadurch gekennzeichnet, daß von dem Eingangskanal (38) eine Leitung (43) abzweigt, die mit einer Pumpe verbunden ist.

20. Analysenvorrichtung nach Anspruch 19, dadurch gekennzeichnet, daß in der Leitung (43) ein Ventil (42) angeordnet ist.

21. Analysenvorrichtung nach Anspruch 12, dadurch gekennzeichnet, daß die Membran (6) ein immobilisiertes Enzym enthält.
